# EUROPEAN PATENT SPECIFICATION

(11) **EP 4 115 831 B1**
(45) Date of publication and mention of the grant of the patent: **23.09.2026**
(21) Application number: 22155797.8
(22) Date of filing: 09.02.2022
(51) Int. Cl.: A61B 18/14, A61B 18/00, A61B 90/00

(54) **IRREVERSIBLE ELECTROPORATION AND THERMAL ABLATION BY FOCAL CATHETER**
IRREVERSIBLE ELEKTROPORATION UND THERMISCHE ABLATION DURCH EINEN FOKALEN KATHETER
ÉLECTROPORATION IRRÉVERSIBLE ET ABLATION THERMIQUE PAR CATHÉTER FOCAL

(30) Priority: 09.07.2021 US 202163220252 P; 07.01.2022 US 202217570989
(43) Date of publication of application: 11.01.2023
(73) Proprietor: Biosense Webster (Israel) Ltd., Yokneam 2066717 (IL)
(72) Inventor: RAO, Anand, Irvine, 92618 (US); DATTA, Keshava, Irvine, 92618 (US); NGUYEN, Thanh, Irvine, 92618 (US)
(74) Representative: Carpmaels & Ransford LLP

(56) References cited:
- EP-A1- 3 248 561
- WO-A1-2021/127558
- US-A1- 2013 030 430
- US-A1- 2014 066 913
- US-A1- 2021 169 550

## Description

### FIELD

The present invention relates to ablation using irreversible electroporation, and particularly a catheter for combined irreversible electroporation and radiofrequency ablation treatments and methods of constructing and using the same.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another. The ablation process destroys the unwanted electrical pathways by formation of non-conducting lesions.

A thermal ablation technique utilizing radio frequency (RF) electrical current is presently the most common ablation technique. Typically, for an ablation procedure using a thermal technique, the electropotentials and the temperatures are measured before, during, and after the actual ablation. When utilizing thermal ablation techniques, care must be taken to avoid thermal damage to non-target tissues and organs which can be heated or cooled during treatment of a target area. Several catheters and probes have been designed to provide RF ablation. One example probe suitable for intracardiac RF ablation is described in U.S. Patent No. 10,660,574.

Ablation by irreversible electroporation (IRE), referred throughout this disclosure interchangeably as pulsed electric field (PEF) ablation and pulsed field ablation (PFA) is a more recently developed technique in which electric potential (voltage) is applied across target tissue which disrupts cellular structures of myocardium and cause cell death. Heat generated during IRE ablation is substantially lower than a comparable RF ablation treatment as IRE ablation does not rely on heat to cause cell death. Some example probes, catheters, and systems which have been developed to provide IRE ablation are described in U.S. Patent Nos. 9,078,665, 9,314,620, and 9,931,161, and U.S. Patent Pub. No. 2014/0296844. Some additional example probes, catheters, and systems which have been developed to provide IRE ablation are described in U.S. Patent Nos 10,188,449 and 10,258,406.

In WO2021127558A1, there is described devices, systems and methods for treating conditions of the heart, particularly the occurrence of arrhythmias.

In EP3248561A1, there is described an electrosurgical generator that includes: a power supply configured to output DC power; an inverter coupled to the power supply, the inverter including a plurality of switching elements; and a controller coupled to the inverter and configured to signal the inverter to simultaneously generate based on the DC power a radio frequency heating waveform and an electroporation waveform.

In US2013030430A1, there is described a medical system, including a medical device having a plurality of deployable arms, and at least one electrode on at least one of the plurality of arms; and an electric signal generator in communication with the medical device, the electric signal generator programmed to deliver pulsed energy to the medical device sufficient to induce irreversible electroporation ablation.

In US2014066913A1, there is described a system that includes a medical device having a plurality of electrodes at a distal end, and an energy generator in communication with the plurality of electrodes, the generator programmable to deliver alternating current energy between approximately 100 volts RMS and approximately 2000 volts RMS or greater. The generator is further programmable to deliver energy in unipolar mode, bipolar mode, and a combination thereof.

In US2021169550A1, there is described an irreversible electroporation and radio frequency ablation (IRE/RFA) generator that includes an IRE pulse generator, harmonic filtration circuitry, and a waveform interleaver.

### SUMMARY

The invention is defined by the appended claims.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, systems, and methods by way of example only, not by way of limitation.
Figure 1 is an illustration of a distal portion of an example catheter according to aspects of the present invention.
Figure 2 is an illustration of an example ablation system being using to treat a patient according to aspects of the present invention.
Figures 3A and 3B are illustrations of an interleaved RF and IRE signal according to aspects of the present invention.
Figure 4 is an illustration of additional interleaved RF and IRE signals according to aspects of the present invention.
Figures 5A and 5B are illustrations of example IRE signals according to aspects of the present invention.
Figure 6 is an illustration of an example method of treatment according to aspects of the present invention.

### DETAILED DESCRIPTION

Although example embodiments of the disclosed technology are explained in detail herein, it is to be understood that other embodiments, within the scope of the appended claims are contemplated. Accordingly, it is not intended that the disclosed technology be limited in its scope to the details of construction and arrangement of components set forth in the following description or illustrated in the drawings. The disclosed technology is capable of other embodiments and of being practiced or carried out in various ways. Features of embodiments disclosed herein, including those disclosed in the attached Appendix of priority application U.S. 63/220,252 can be combined as understood by a person skilled in the pertinent art according to the teachings as defined by the appended claims.

It must also be noted that, as used in the specification and the appended claims, the singular forms "a," "an" and "the" include plural referents unless the context clearly dictates otherwise. By "comprising" or "containing" or "including" it is meant that at least the named system, apparatus, device, component, compound, element, particle, or method or use step is present in the system or apparatus or method or use, but does not exclude the presence of other systems, apparatuses, devices, components, compounds, materials, particles, method or use steps, even if the other such systems, apparatuses, devices, components, compounds, material, particles, method or use steps have the same function as what is named.

As used herein, the terms "about" or "approximately" for any numerical values or ranges indicate a suitable dimensional tolerance that allows the part or collection of components to function for its intended purpose as described herein. More specifically, "about" or "approximately" can refer to the range of values ±20% of the recited value, e.g. "about 90%" can refer to the range of values from 71% to 99%.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, or any other individual or delivery instrumentation associated with delivery of a multi-electrode balloon catheter for the treatment of drug refractory atrial fibrillation to a subject.

As discussed herein, the term "ablate" or "ablation", as it relates to the devices and corresponding systems of this disclosure, refers to components and structural features which are configured to reduce or prevent the generation of erratic cardiac signals. Non-thermal ablation includes use of irreversible electroporation (IRE) to cause cell death, referred throughout this disclosure interchangeably as pulsed electric field (PEF) and pulsed field ablation (PFA). Thermal ablation includes use of extreme temperature to cause cell death and includes RF ablation. Ablating or ablation as it relates to the devices and corresponding systems of this disclosure is used throughout this disclosure in reference to ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" as it generally relates to known methods, devices, and systems includes various forms of bodily tissue ablation as understood by a person skilled in the pertinent art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective components. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a "square shape" having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "pulse train" and "train" equivalently refer to a sequence of biphasic or monophasic pulses distributed in time such that the pulses are separated by a short delay. Pulses in a pulse train are delivered a pulse frequency that is an inverse of the period of a pulse in the pulse train. For biphasic pulse trains, the delay is preferably approximately equal to the interphase delay between phases in a single biphasic pulse of the pulse train.

As discussed herein, the terms "pulse burst" and "burst" equivalently refer to a sequence of pulse trains distributed in time such that pulse trains are separated by regular time intervals. Preferably the pulse trains in the burst are distributed in time such that the termination of the last phase of a leading train is separated in time from an initiation of a trailing train by an inter-pulse delay significantly longer in duration than the delay between pulses in the pulse train.

As discussed herein, the term "lesion" is intended to cover damage to tissue caused by the ablation electrodes sufficient to substantially reduce or eliminate erratic cardiac signals propagating through the damaged tissue. It is noted that damages to tissue under the term "lesion" are intended to cover any tissue damage, including thermal and non-thermal, some of which may not be visible but can be detected by a suitable modality and up to visible tissue damage.

As discussed herein, the term "processor" may include one or more processing units (e.g., in a multi-core configuration). Further, processors described herein may be implemented using one or more heterogeneous processor systems in which a main processor is present with secondary processors on a single chip. As another illustrative example, the processors may be a symmetric multi-processor system containing multiple processors of the same type. Further, the processors may be implemented using any suitable programmable circuit including one or more systems and microcontrollers, microprocessors, reduced instruction set circuits (RISC), application specific integrated circuits (ASIC), programmable logic circuits, field programmable gate arrays (FPGA), and any other circuit capable of executing the functions described herein. Additionally, the processors may perform partial processing and receive partial processing by a processor and/or computing device communicatively coupled to the processors.

As discussed herein, the term "non-transitory computer-readable media" includes, but is not limited to, random access memory (RAM), read-only memory (ROM), electronically erasable programmable ROM (EEPROM), flash memory or other memory technology, compact disc ROM (CD-ROM), digital versatile disks (DVD) or other optical storage, magnetic cassettes, magnetic tape, magnetic disk storage or other magnetic storage devices, or any other tangible, physical medium which can be used to store computer readable information.

The present disclosure is related to systems and non-claimed method or uses and devices for ablating cardiac tissue to treat cardiac arrhythmias. Ablative energies can be provided to cardiac tissue by a distal portion of a catheter which can deliver ablative energy alongside the tissue to be ablated. Example catheters presented herein are configured provide electrical energy for ablation with IRE and have a form factor that is generally similar to catheters currently used to perform RF ablation. The catheter is further configured to delivery RF electrical current for thermal ablation simultaneously with ablation by IRE

RF catheter ablation is presently the dominant modality for ablative therapy of arrhythmias. When ablating tissue using RF energy, most of the tissue heating resulting in the formation of lesion occurs as a result of thermal conduction from the direct resistive heat source, i.e. electrode(s) delivering RF energy. Transfer of heat through tissue follows basic thermodynamic principles, and the boundary of the lesion is generally determined by a 50°C isotherm.

Ablation with RF energy is generally considered to have a high success rate and low complication rate. However, RF approaches can have a risk of thermal cell injury to the patient which can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However, maneuvering cryoablation devices and selectively applying cryoablation is generally more challenging compared to RF ablation; therefore, cryoablation is not viable in certain anatomical geometries which may be reached only by electrical ablation devices.

Electroporation can be induced by applying a pulsed electric field across biological cells to cause reversable (temporary) or irreversible (permanent) creation of pores in the cell membrane. Cells have a transmembrane electrostatic potential that is increased above a resting potential upon application of the pulsed electric field. While the transmembrane electrostatic potential remains below a threshold potential, the electroporation is reversable, meaning the pores can close when the applied electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas bone has a threshold potential of approximately 3000 V/cm. These differences in threshold potential allow IRE to selectively target cells based on threshold potential.

Ablation with IRE is a non-thermal cell death technology that can be used for ablation of atrial arrhythmias that reduces or eliminates thermal risks associated with RF ablation. To ablate using IRE/PEF, biphasic or monophasic voltage pulses are applied to disrupt cellular structures of myocardium. The pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE has the capability to spare adjacent heat sensitive structures or tissues which would be of benefit in the reduction of possible complications known with ablation or isolation modalities. Example systems and catheters configured to both IRE and RF ablation can therefore provide a multi-functional tool for ablation treatments.

Example systems and catheters can also be configured to induce reversible electroporation as part of a diagnostic procedure. Reversible electroporation can temporarily interrupt electrical activation signals through targeted myocardial tissue to allow for observation of changes in electrical activity within the heart which can be used to predict the outcome of ablation at the targeted tissue. Temporarily interrupted electrical activation signals induced through reversible electroporation can resume as the cells self-repair.

The pulsed electric field, and its effectiveness to induce reversible and/or irreversible electroporation, can be affected by physical parameters of the system and pulse parameters of the electrical signal. Physical parameters can include electrode contact area, electrode spacing, electrode geometry, etc. Pulse parameters of the electrical signal can include voltage amplitude, pulse duration, pulse interphase delay (biphasic pulses), inter-pulse delay, total application time, delivered energy, etc. In some examples, parameters of the electrical signal can be adjusted to induce both reversible and irreversible electroporation given the same physical parameters.

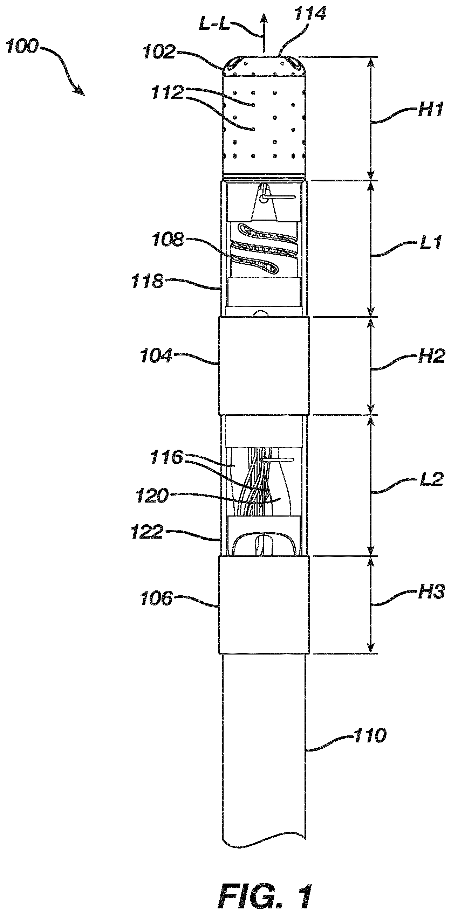

Figure 1 is an illustration of a distal portion of an example catheter 100 configured to provide electrical energy to ablate using both RF and IRE. The catheter 100 can further be configured to induce reversible electroporation. The catheter 100 includes a tip electrode 102, a middle ring electrode 104, and a proximal ring electrode 106. The electrodes 102, 104, 106 can be configured to ablate using RF and IRE in an interleaved pattern and/or ablate using RF and IRE simultaneously. The electrodes 102, 104, 106 are aligned linearly with a longitudinal axis L-L of the catheter 100.

In some examples, any of the electrodes 102, 104, 106 can be configured to ablate using both RF and IRE, meaning the electrodes are spaced, sized, and otherwise constructed to deliver electrical currents and voltages to perform both RF and IRE ablation. In such examples, the application of electrical signals to the electrodes 102, 104, 106 to perform RF or IRE ablation can be selected for a particular treatment. Alternatively, any of the electrodes 102, 104, 106 can be configured to ablate using RF or IRE but not both. The catheter can include additional ring electrodes (not illustrated) configured for RF and/or IRE ablation. Electrodes configured to perform RF ablation can be configured to provide a sufficient contact area against tissue to facilitate heat transfer to tissue. The catheter 100 can be configured for IRE ablation by being configured to withstand biphasic pulse bursts having an amplitude of about 200 V to about 2,000 V. Preferably, the catheter 100 can be configured such that pulses between alternative electrodes can have an amplitude about twice an amplitude of pulses between adjacent electrodes. In one example, the catheter 100 can be configured for IRE ablation by being configured to withstand biphasic pulse bursts having an amplitude of about 900 V between adjacent electrodes and 1800 V between alternate electrodes. The maximum voltage amplitude that the catheter 100 is configured to withstand can be dependent on electrode length, electrode diameter, electrode spacing, size of return electrode, interior dimensions of conductors within the catheter, insulation between conductors within the catheter, etc. as understood by a person skilled in the pertinent art.

The tip electrode 102 is shaped as a dome at a distal end 114 of the catheter 100. The tip electrode 102 includes irrigation ports 112 configured to provide irrigation fluid to regulate tissue temperature during ablation. Because IRE ablation does not rely on thermal damage to create lesions irrigation may not be necessary to regulate tissue temperature in some treatments; therefore, irrigation ports 112 and related structures (e.g. irrigation tube 120) can be omitted for an example catheter configured for such treatments.

The tip electrode 102 has a height H1 measured along a longitudinal axis L-L of the catheter 100 preferably of about 3.5 millimeters (mm).

The tip electrode can include two electrode halves (not illustrated) electrically insulated from each other. Each half can be disposed over a respective portion of the distal end of the catheter. At least one of the halves can be configured to provide RF ablation electrical signals.

The tip electrode 102 can further include thermocouples and other sensors and otherwise be configured similar to corresponding structures in U.S. Patent No. 10,660,574.

The catheter 100 can further include a contact force sensor 108 surrounded by a flexible sheath 118 configured similarly to corresponding structures in U.S. Patent No. 10,660,574, variations thereof, and alternatives thereto as understood by a person skilled in the pertinent art. The sheath 118 is illustrated as transparent solely for the purpose of illustration. The contact force sensor 108 can be configured to sensor force applied to the tip electrode 102.

The ring electrodes 104, 106 are positioned in a proximal direction in relation to the tip electrode 102 around the tubular body of the catheter 100. Each ring electrode 104, 106 respectively has a height H2, H3 measured along the longitudinal axis L-L of preferably about 3 mm. The middle electrode 104 is spaced a length L1 measuring edge to edge from the tip electrode 102 of preferably about 4 mm. The proximal electrode 106 is spaced a length L2 measuring edge to edge from the middle electrode 104 of preferably about 4 mm. Although not illustrated, either or both of the ring electrodes 104, 106 can optionally include irrigation ports and can be configured to provide irrigation to regulate tissue temperature during treatment.

The catheter 100 can include wires and cables 116 configured to carry electrical signals to the electrodes 102, 104, 106, contact force sensor 108, and other electronic components. The catheter 100 can include a support tube 122 between the middle ring electrode 104 and proximal ring electrode 106 illustrated as transparent for the sake of illustration.

The catheter 100 has an elongated shaft 110 that can be manipulated to position the distal portion at a treatment site, for instance within or adjacent a heart in a living patient 24. Preferably, and without limitation, the catheter 100 can have a shaft size having an outer diameter (OD) of approximately 7.6 French (Fr), a sheath compatibility of approximately 8.5 Fr or greater, electrodes constructed with platinum-iridium, and a bi-directional deflection. However, the catheter 100 is not so limited and can vary to meet needs of a specific use. The catheter 100 can be sized and otherwise configured to introduced through the femoral artery, wrist artery (radial access), directly through the carotid artery, or other approach as known to a person skilled in the pertinent art.

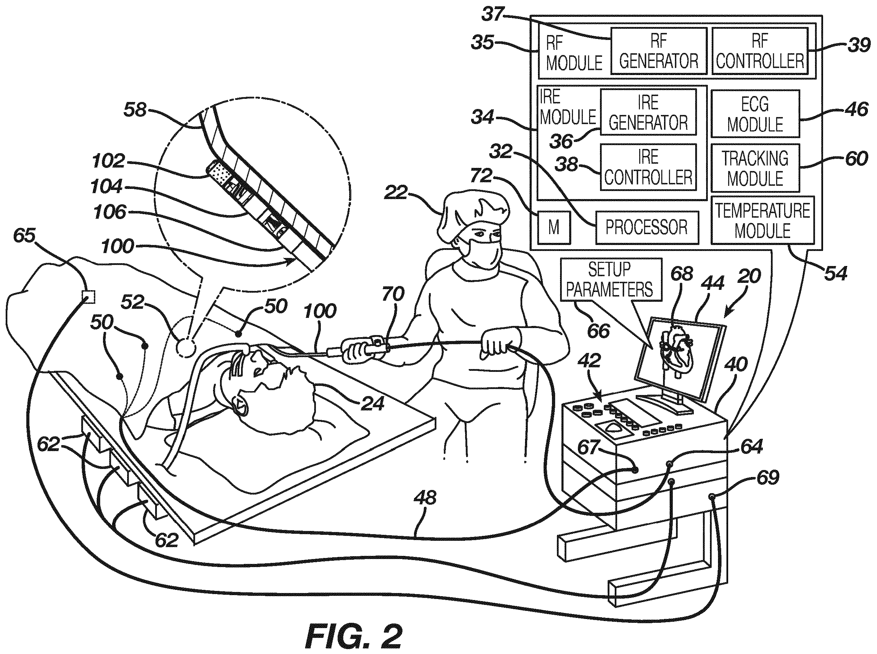

Figure 2 is an illustration of an example system 20 configured to utilize the catheter 100 in an example illustrated ablation procedure. A handle 70 is used by a physician 22 to move the distal portion of the catheter 100 to a treatment site. Some or all of the electrodes 102, 104, 106 can be positioned against tissue 58 at the treatment site. Contact force can be measured with a force sensor.

IRE is a predominantly non-thermal process, which causes an increase of the tissue temperature by, at most, a few degrees for a few milliseconds. It thus differs from RF (radio frequency) ablation, which raises the tissue temperature by between 20 and 70°C and destroys cells through heating. IRE can utilize biphasic pulses, i.e., combinations of positive and negative pulses, in order to avoid muscle contraction from a DC voltage. The IRE pulses, alone or in combination with RF ablation, can be generated and applied in various treatments such as described in U.S. Patent Pub. No. 2021/0169550, U.S. Patent Pub. No. 2021/0169567, U.S. Patent Pub. No. 2021/0169568, U.S. Patent Pub. No. 2021/0161592,, U.S. Patent Pub. No. 2021/0186604. U.S. Patent Application No. 16/989,445 claims priority to U.S. 62/949,999 and is published as U.S. Patent Pub. No. 2021/0191642. U.S. Patent Application No. 16/731,238 is published as U.S. Patent Pub. No. 2021/0196372. Application No. 16/710,062 is published as U.S. Patent Pub. No. 2021/0177503.

The pulses of IRE ablation can be applied between two electrodes 102, 104, 106 of the catheter 100 in a bipolar mode or can be applied between a catheter electrode 102, 104, 106 and a body patch 65 in a unipolar mode. Applied voltages during IRE ablation may reach up to 2000 V, which is much higher than the typical voltage of 10-200 V in thermal RF ablation. Signals applied during RF ablation can be applied between one or more of the electrodes 102, 104, 106 and the same or different body patch 65.

The illustrated ablation system 20 includes a processor 32, an IRE module 34, an RF module 35, an electrocardiogram (ECG) module 46, a tracking module 60, and a temperature module 54. The modules 34, 35, 46, 60, 54 can, collectively or individually, include non-transitory memory with instructions thereon that can be executed by the processor to perform various functions including those described herein and those understood by a person skilled in the pertinent art according to the teachings herein.

The IRE module includes an IRE generator 36 and an IRE controller 38. The RF module 35 includes an RF generator 37 and an RF controller 39. The RF controller 39 and IRE controller 38 can be integral or configured to work in concert, for instance to generate interleaved waveforms similar to as disclosed in U.S. Patent Pub. No. 2021/0169550, as disclosed in U.S. Patent Pub. No. 2021/0161592, or other waveform patterns as disclosed herein including those disclosed in the attached Appendix of priority application U.S. 63/220,252. The ablation system 20 can be configured to provide simultaneous and/or sequential RF and IRE signals to the electrodes 102, 104, 106.

The RF module 35 and IRE module 34 are illustrated as separate modules for the sake of illustration, and it is to be understood that the modules can share common hardware and software components. The processor 32, IRE controller 38, and RF controller 39 each can include a programmable processor, which is programmed in software and/or firmware to carry out the functions that are described herein. Alternatively, or additionally, the IRE controller 38, RF controller 39, and/or processor 32 can each include hard-wired and/or programmable hardware logic circuits, which carry out at least some of these functions. Although the processor 32 is illustrated as a functional block separate from the IRE controller 38 and RF controller 39, in practice some of these functions may be combined in a single processing and control unit, with suitable interfaces for receiving and outputting the signals that are illustrated in the figures and are described in the text. For instance, the IRE controller 38 can reside within the IRE module 34, as high-speed control signals are transmitted from the IRE controller to the IRE generator 36. However, provided that signals at sufficiently high speeds may be transmitted from the processor 32 to the IRE generator 36, the IRE controller 38 can reside within the processor.

The processor 32 and the IRE module 34 can reside within the console 40. The console 40 includes input devices 42, such as a keyboard and a mouse. A display screen 44 is located in proximity to (or integral to) console 40. The display screen 44 can optionally include a touch screen, thus providing another input device.

The electrocardiogram (ECG) module 46, the temperature module 54, and/ or the tracking module 60 can reside within the console 40 and can be connected to suitable interfaces and devices in the system 20. As illustrated, the electrocardiogram (ECG) module 46 is coupled through a cable 48 to ECG electrodes 50, which are attached to the subject 24. The ECG module 46 is configured to measure the electrical activity of a heart 52 of the subject 24. The temperature module 54 is coupled to optional temperature sensors, such as thermocouples within the distal tip electrode 102 of the catheter 100. The tracking module 60 is coupled to one or more electromagnetic position sensors (not shown) in the distal portion of the catheter 100. In the presence of an external magnetic field generated by one or more magnetic field generators 62, the electromagnetic position sensors output signals that vary with the positions of the sensors. Based on these signals, the tracking module 60 may ascertain the positions of electrodes 102, 104, 106 in the heart 52. The modules 46, 54, 60 typically include both analog and digital components, and are configured to receive analog signals and transmit digital signals. Each module can additionally include hard-wired and/or programmable hardware logic circuits, which carry out at least some of the functions of the module.

The catheter 100 is coupled to the console 40 via an electrical interface 64, such as a port or socket. IRE signals and RF signals are thus carried to the distal portion of the catheter 100 via the console interface 64. Similarly, signals for tracking the position of the electrodes 102, 104, 106, and/or signals for tracking the temperature of tissue 58, may be received by the processor 32 via the interface 64 and applied by the IRE controller 38 in controlling the pulses generated by the IRE generator 36 and/or applied by the RF controller 39 in controlling RF signals generated by the RF generator 37.

One or more external electrodes 65, or "return patches", can be coupled externally to the subject 24, typically on the skin of the subject's torso and the console 40 via a body patch port 69. The return patch(es) 65 can provide a return path (return paths) for unipolar ablation signals applied at one or more of the ablation electrodes 102, 104, 106 of the catheter 100. RF ablation and IRE ablation can each be applied in a unipolar ablation scheme. Further, RF ablation and IRE ablation can each be applied in a bipolar ablation scheme. Presently it is more common for RF ablation to be performed in a unipolar ablation scheme and for IRE to be performed in a bipolar ablation scheme. The example catheter 100 and the example system 20 can be adapted to perform unipolar RF ablation, unipolar IRE ablation, bipolar RF ablation, unipolar IRE ablation, or any combination thereof.

During unipolar RF ablation, RF energy is delivered between at least one of the electrodes 102, 104, 106 of the catheter 100 and the return patch 65. When multiple catheter electrodes 102, 104, 106 are activated for unipolar RF ablation, the electrodes can be electrically synchronized or electrically tied together to inhibit current flow between activated electrodes. The electrical alternating current flowing through the tissue encounters resistance, and the energy is converted into heat. This energy, resistive heat, destroys the tissue close to the activated catheter electrode(s) 102, 104, 106. Heat is then transmitted to the surrounding tissue by conduction and radiation leading to lesion formation. Scar formation is related to the electrode size, power of the energy, the contact force between the electrode and the tissue and the cooling effect of the surrounding fluid, even though only a small amount of the energy is delivered to the tissue. Cooling the electrode can reduce the risk of overheating the tissue thereby enabling creation of a deeper lesion. Decrease of distance between the electrode(s) 102, 104, 106 and the return patch 65 can result in a bigger current density with more resistive heating, which leads to deeper lesion formation. Bipolar RF ablation is when RF current flows between two of the catheter electrodes 102, 104, 106. Lesions created by bipolar RF ablation are generally narrower and deeper than lesions created by unipolar RF ablation, resulting in a transmural scar in the tissue as thick as about 25 mm.

During bipolar IRE ablation, biphasic pulses are applied between catheter electrodes 102, 104, 106 (typically in pairs) to create an electric field between the electrodes 102, 104, 106. Cells at and between the electrodes experience the greatest electric fields and undergo electroporation. During unipolar IRE ablation, biphasic pulses are applied between one or more catheter electrodes 102, 104, 106 and the return patch(es) 65 to create an electric field between the distal portion of the catheter 100 and the return patch(es). When multiple catheter electrodes 102, 104, 106 are activated for unipolar IRE ablation, the electrodes can be electrically synchronized or electrically tied together to inhibit current flow between activated electrodes. Electric flux density is greatest at the activated electrodes and therefore a higher density of cells experience electroporation at the electrodes. Unipolar IRE ablation may be useful in certain treatments as the state of the art advances, for instance ablating thicker cardiac tissues in the ventricle.

The processor 32 is configured to receive setup parameters 66 from the physician 22 or another user or device. Using one or more suitable input devices 42, the physician 22 can input the parameters of ablation signals for RF ablation and/or IRE ablation. The physician 22 may select ablation electrodes 102, 104, 106 for activation (for receiving the IRE pulses and/or RF signals) and the order in which they are activated. In setting up the ablation, the physician 22 may also choose a mode of synchronization of the IRE pulses with respect to the cycle of heart 52.

In response to receiving setup parameters 66, the processor 32 communicates these parameters to the IRE controller 38 and RF controller 39, which commands the IRE generator 36 and RF generator 37 to generate IRE signals and RF signals respectively in accordance with the setup requested by physician 22. Additionally, the processor 32 may display setup parameters 66 on display screen 44.

The processor 32 can be configured to display on the display 44, based on signals received from the tracking module 60, a relevant image 68 of the subject's anatomy, annotated, for example, to show the current position and orientation of the electrodes 102, 104, 106. Alternatively, or additionally, based on signals received from the temperature module 54 and the ECG module 46, the processor 32 may display on the display screen 44 the temperatures of tissue 58 and electrical activity of heart 52.

To begin the procedure, physician 22 inserts the catheter 100 into a subject 24, and then navigates the catheter, using a control handle 70, to an appropriate site within, or external to, heart 52. Subsequently, physician 22 brings some or all of the electrodes 102, 104, 106 into contact with tissue 58, such as myocardial or epicardial tissue, of heart 52. Next, ablation signals are applied to some or all of the electrodes 102, 104, 106.

In some treatments, a treatment site can be ablated using IRE ablation via the electrodes 102, 104, 106. Once bipolar IRE ablation is complete, the distal tip electrode 102 can be used to perform RF ablation for touch ups to areas of the treatment site that were not ablated by IRE ablation (e.g. carina).

The processor 32 (which can be a single processor or multiple processors) can be in communication with non-transitory computer readable medium 72 (referred to hereinafter as "memory" for the sake of brevity) with instructions thereon that can be executed by the processor 32 to cause the ablation system console 40 to perform various functions. The memory 72 can include one or more separate hardware components, can be positioned within the console 40, fixed within the console 40, removable from the console 40, and/or remote from the console (e.g. accessible by the console 40 and providing software as a service). Preferably, the memory 72 is fixed within the console 40 and the various modules 34, 35, 46, 54, 60 of the console 40 are represented as software modules of the memory 72. Alternatively, some or all of the modules 34, 35, 46, 54, 60 can include a dedicated processor and/or memory that is electrically segregated from other modules. As discussed herein, the terms "processor" and "memory" when referring to the console 40 includes a variety of physical manifestations as understood by a person skilled in the pertinent art and reference to "processor" and "memory" in a singular form is intended to include non-singular manifestations unless otherwise made apparent.

During an example treatment, instructions on the memory 72 can be executed by the processor 32 to cause the console 40 to provide an RF ablation signal via the output port 64 (which can include multiple output ports) to one of the catheter electrodes 102, 104, 106 and provide an IRE ablation signal via the output port 64 to at least one of the other catheter electrodes 102, 104, 106. The IRE ablation signal can include monophasic pulses and/or biphasic pulses and preferably is primarily biphasic to avoid application of DC voltage. The IRE ablation signal can be bipolar or unipolar and is preferably bipolar to avoid skeletal and muscular pain. The RF ablation signal can be unipolar or bipolar. Although unipolar RF ablation is currently more common than bipolar RF ablation, bipolar RF ablation may have some benefits as discussed elsewhere herein.

Continuing with the example treatment, the instructions on the memory 72 can be executed by the processor 32 to cause the console 40 to provide the IRE ablation signal as a burst of biphasic pulses between the body patch 65 via the body patch port 67 and at least one of the catheter electrodes 102, 104, 106 via the output port 64. Alternatively, and not requiring the body patch port 69, the instructions on the memory 72 can be executed by the processor 32 to cause the console 40 to provide the IRE ablation signal via the output port 64 as a burst of biphasic pulses between two of the catheter electrodes 102, 104, 106. As another alternative, when the console 40 includes the body patch port 69, the instructions on the memory 72 can be executed by the processor 32 to cause the console 40 to provide IRE ablation signals between the body patch 65 and at least one of the catheter electrodes 102, 104, 106 and also provide IRE ablation signals between two of the catheter electrodes 102, 104, 106.

The instructions on the memory 72 can be executed by the processor 32 to cause the console 40 to provide the burst of biphasic pulses between two of the catheter electrodes 102, 104, 106 via the output port 64 while simultaneously providing the RF ablation signal to the remaining catheter electrode 102, 104, 106 via the output port 64. In this case, the biphasic pulses are bipolar between the two catheter electrodes 102, 104, 106 to provide bipolar IRE ablation. The RF ablation signal can be provided to the tip electrode 102, thereby providing unipolar RF ablation using the return patch 65, while the burst of biphasic pulses is provided between the ring electrodes 104, 106, thereby providing bipolar IRE ablation. Alternatively, or subsequently, the RF ablation signal can be provided to one of the ring electrodes (e.g. the proximal ring electrode 106), thereby providing unipolar RF ablation, while the burst of biphasic pulses is provided between the remaining ring electrode (e.g. the middle ring electrode 104) and the tip electrode 102, thereby providing bipolar IRE ablation.

The instructions on the memory 72 can be executed by the processor 32 to cause the console 40 to provide a first IRE ablation signal via the output port 64 as a first burst of biphasic pulses between two of the catheter electrodes functioning as a first pair of electrodes and provide a second IRE ablation signal via the output port(s) as a second burst of biphasic pulses between two catheter electrodes functioning as a second pair of electrodes having one electrode in common with the first pair of electrodes and one electrode not in common with the first pair of electrodes. For instance, the first pair of electrodes can include the middle ring electrode 104 and the proximal ring electrode 106 and the second pair of electrodes can include the tip electrode 102 and the proximal ring electrode 106. The instructions on the memory 72 can be executed by the processor to cause the console to provide the first burst of biphasic pulses at an amplitude of about 900 V and provide the second burst of biphasic pulses at an amplitude of about 1800 V. A third IRE ablation signal can be applied between the tip electrode 102 and the middle ring electrode 104. The amplitude of the third IRE ablation signal can be about 900 V. The first IRE ablation signal, second IRE ablation signal, and third IRE ablation signal can be performed in any order to form a triplet. The first, second, and third IRE ablation signals can each include pulse trains that are synchronized with each other to repeat the triplet pattern for the duration of the respective pulse trains.

The instructions on the memory 72 can be executed by the processor to cause the console to provide one or more RF ablation signals before and/or after the IRE ablation signals, interleaved with the IRE ablation signals and/or simultaneously with the IRE ablation signals. For instance, the console can simultaneously provide an IRE ablation signal and an RF ablation signal via the output port 64, then at a later time, simultaneously provide a subsequent IRE ablation signal and a subsequent RF ablation signal via the output port 64 such that the electrode providing RF ablation and the electrode pair providing IRE ablation changes when the subsequent ablation signals are applied.

In examples where the tip electrode 102 has two electrically isolated halves, the instructions on the memory 72 can be executed by the processor 32 to cause the console 40 to provide the RF ablation signal to a first half of the tip electrode 102 and not to a second half of the tip electrode.

In examples where the catheter 100 includes a force sensor 108, the instructions on the memory 72 can be executed by the processor 32 to cause the console 40 to determine a force vector applied to the distal portion of the catheter 100 based on a sensor signal received by the console 40 from the catheter 100.

The ablation system console can further include an irrigation module (not illustrated) configured to provide irrigation fluid to the first catheter electrode. The console 40 and system 20 can be modified to include irrigation as understood by a person skilled in the pertinent art.

The instructions on the memory 72 can be executed by the processor to cause the console 40 to determine temperature of a catheter electrode 102, 104, 106 configured to provide RF ablation (e.g. tip electrode 102) based at least in part on a thermal sensor signal received from the catheter 100.

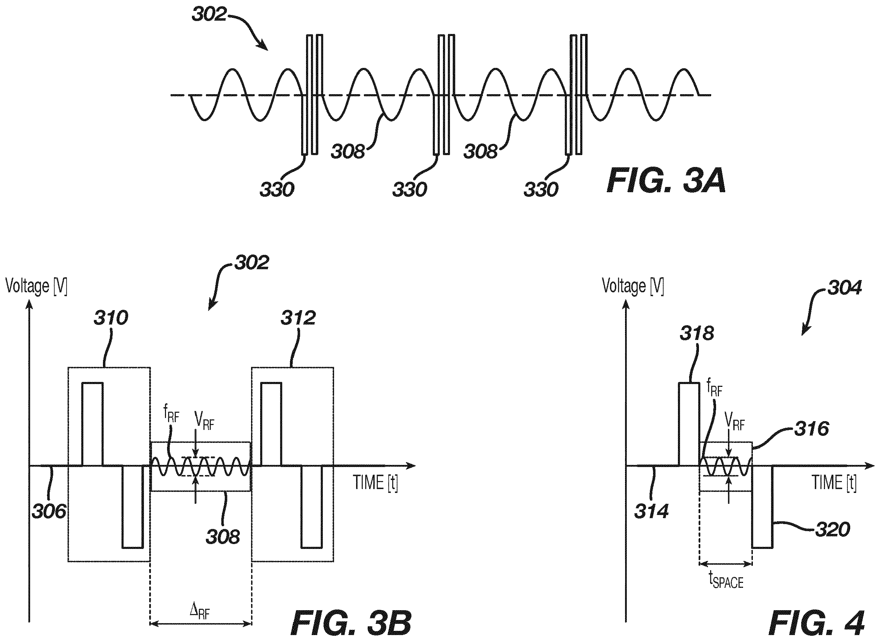

Figures 3A and 3B are illustrations of an interleaved RF and IRE signal 302. Figure 3B is a view of the signal 302 illustrated in Figure 3A on a shorter timescale so that features of the signal 302 can be visualized in greater detail. The signal 302 can be generated as described in greater detail in U.S. Patent Pub. No. 2021/0169550 and U.S. Patent Pub. No. 2021/0161592.

As illustrated in Figure 3A, the interleaved signal 302 can include sinusoidal RF signals 308 interleaved between pulse trains 330. The pulse trains 330 are illustrated as including two biphasic pulses; however, the pulse trains 330 can include additional biphasic pulses to achieve a desired treatment outcome. The interleaved signal 302 can be applied to perform unipolar and/or bipolar ablation. For instance, the interleaved signal 302 can be applied between the tip electrode 102 and the reference patch 65 (Figure 2) to provide unipolar IRE ablation and RF ablation. As another example, the interleaved signal 302 can be applied between the ring electrodes 104, 106 to provide bipolar IRE and RF ablation. As another example, the interleaved signal 302 can be applied to the tip electrode while a switch toggles the return path for the interleaved signal 302 between the reference patch 65 and a ring electrode 104, 106 to provide unipolar RF ablation and bipolar IRE ablation. Building on this logic, the interleaved signal 302 can be applied to numerous combinations of the electrodes 102, 104, 106 to achieve various combinations of unipolar and bipolar ablation schemes as understood by a person skilled in the pertinent art.

As illustrated in Figure 3B, a curve 306 depicts the voltage V as a function of time t of an RF signal 308 between two bipolar pulses 310, 312 which are from pulse trains 330 illustrated in Figure 3A. The amplitude of the RF signal 308 is labeled as V_{RF}, its frequency is labeled as f_{RF}, and the separation between the pulses 310, 312 (and thereby the pulse trains 330) is labeled as Δ_{RF}. Preferably, the frequency f_{RF} is between about 350 kilohertz (kHz) and about 500 kHz, but higher or lower frequencies may be alternatively used.

Figure 4 is an illustration of another example interleaved RF and IRE signal 304 having a curve 314 with an RF signal 316 between a positive phase 318 and a negative phase 320 of a biphasic pulse. An interphase delay t_{SPACE} between the positive phase 318 and negative phase 320 can be stretched compared to the biphasic pulses 310, 312 illustrated in Figures 3A and 3B to accommodate the RF signal between phases 318, 320. The interleaved signal 304 illustrated in Figure 4 can be repeated to generate pulse trains and pulse bursts.

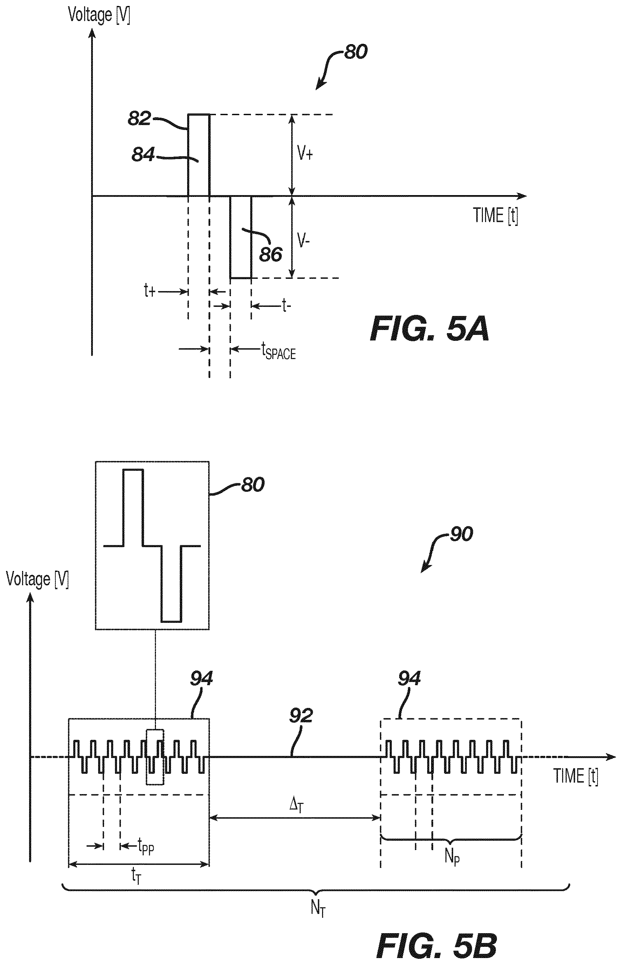

Figure 5A is an illustration of a biphasic pulse 80. A curve 82 depicts the voltage V of biphasic IRE pulse 80 as a function of time t. The pulse 80 includes a positive phase 84 and a negative phase 86. The amplitude of the positive phase 84 is labeled as V+ and the temporal pulse width of the phase is labeled as t+. Similarly, the amplitude of the negative phase 86 is labeled as V- and the temporal pulse width of the phase 86 is labeled as t-. The interphase delay is labeled as t_{SPACE}. Values for the parameters of the pulse 80 can be found in the references attached in the appendix of priority application U.S. 63/220,252, particularly U.S. Patent Pub. No. 2021/0161592 and U.S. Patent No. 16/710,062 (Attorney Docket No. BIO6209USNP1).

Figure 5B is an illustration of a curve 92 of a burst 90 of biphasic pulse trains 94. The burst 90 includes N_{T} pulse trains 94. where each train includes N_{P} biphasic pulses. The length of the pulse train 94 is labeled as t_{PP}, and the interval between consecutive trains is labeled as Δ_{T}. Values for the parameters of the pulse 80 can be found in the references attached in the appendix of priority application U.S. 63/220,252, particularly U.S. Patent Pub. No. 2021/0161592 and U.S. Patent No. 16/710,062 (Attorney Docket No. BIO6209USNP1).

Any of the example signals 302, 304, 90 illustrated in Figures 3A through 5B can be applied in a triplet in which the respective signal 302, 304, 90 is applied in a synchronous and bipolar fashion between three electrode pair combinations: tip electrode 102 to middle ring electrode 104, middle ring electrode 104 to proximal ring electrode 106, and tip electrode 102 to proximal ring electrode 106. Voltage amplitude of at least the IRE pulses can be increased for the pair including the tip electrode 102 and proximal ring electrode 106 given the increased distance between the electrodes in that pair compared to the other pairs.

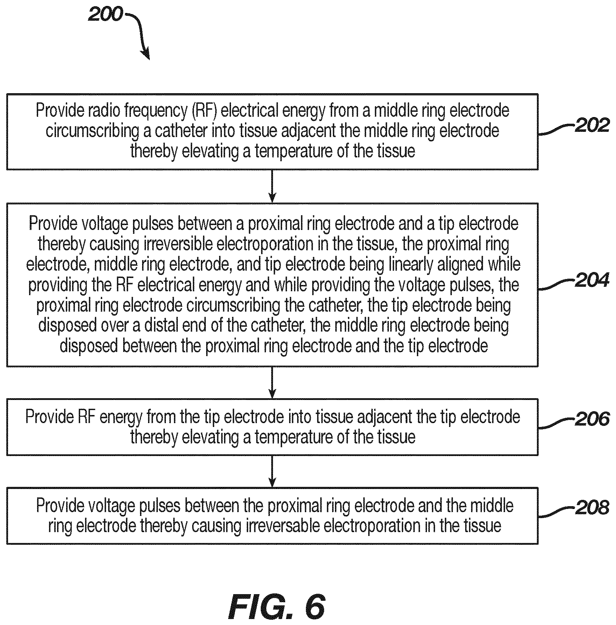

Figure 6 is an illustration of a flow diagram of an example, non-claimed, method 200 of performing ablation. The method 200 can be performed using a catheter 100 and/or system 20 as illustrated in Figures 1 and 2 respectively, variations thereof, and alternatives thereto as understood by a person skilled in the pertinent art.

At step 202, RF electrical energy is provided from a middle ring electrode circumscribing a catheter into adjacent tissue thereby elevating a temperature of the tissue. The middle ring electrode can be configured similarly to the middle ring electrode 104 of the catheter 100 illustrated in Figure 1, a variation thereof, or an alternative thereto as understood by a person skilled in the pertinent art.

At step 204, voltage pulses can be provided between a proximal ring electrode and a tip electrode thereby causing irreversible electroporation in the tissue. The proximal ring electrode, middle ring electrode, and tip electrode being linearly aligned while providing the RF electrical energy and while providing the voltage pulses. The proximal ring electrode can circumscribe the catheter, the tip electrode can be disposed over a distal end of the catheter, and the middle ring electrode can be disposed between the proximal ring electrode and the tip electrode. The proximal ring electrode and tip electrode can be configured similarly to corresponding structures 106, 102 of the catheter 100 illustrated in Figure 1, a variation thereof, or an alternative thereto as understood by a person skilled in the pertinent art.

At step 206, RF energy can be provided from the tip electrode into tissue adjacent the tip electrode thereby elevating a temperature of the tissue.

At step 208, voltage pulses can be provided between the proximal ring electrode and the middle ring electrode thereby causing irreversible electroporation in the tissue.

The method 200 can further include providing voltage pulses between the tip electrode and middle ring electrode in a triplet pattern. The method can further include providing RF electrical energy from the proximal electrode. The steps can be performed in various orders and simultaneously with each other in various combinations thereto as understood by a person skilled in the pertinent art according to the teachings herein. The method 200 can include additional steps to achieve functionality as described elsewhere herein such as measuring force at the catheter tip and/or irrigating through one or more of the electrodes.

## Claims

1. An ablation system console (40) comprising:
one or more output ports (64) configured to provide ablation energy to first (102), second (104), and third (106) catheter electrodes while the first, second and third catheter electrodes are linearly aligned on a longitudinal axis of a distal portion of a catheter (100);
a processor (32); and
non-transitory computer readable medium in communication with the processor and comprising instructions thereon, that when executed by the processor, cause the console to:
provide an RF ablation signal via the output port(s) to the first catheter electrode,
provide an IRE ablation signal via the output port(s) as a burst of biphasic pulses between the second and third catheter electrodes, and **characterized in that** the non-transitory computer readable medium further comprises instructions thereon, that when executed by the processor, cause the console to
provide the burst of biphasic pulses between the second catheter electrode and the third catheter electrode via the output port(s) while simultaneously providing the RF ablation signal to only the first catheter electrode via the output port(s).

2. The ablation system console of claim 1, further comprising:
a body patch port (69),
the non-transitory computer readable medium further comprising instructions thereon, that when executed by the processor, cause the console to:
provide the IRE ablation signal as a burst of biphasic pulses between a body patch (65) via the body patch port and at least one of the second and third catheter electrodes via the output port(s).

3. The ablation system console of claim 1, the non-transitory computer readable medium further comprising instructions thereon, that when executed by the processor, cause the console to:
provide the burst of biphasic pulses and the RF ablation signal via the output port(s) such that the first catheter electrode is configured as a tip electrode disposed over a tip of the catheter and the second and third catheter electrodes are each configured as respective ring electrodes circumscribing a body of the catheter in a proximal direction in relation to the tip electrode.

4. The ablation system console of claim 1, the non-transitory computer readable medium further comprising instructions thereon, that when executed by the processor, cause the console to:
provide the burst of biphasic pulses and the RF ablation signal via the output port(s) such that the second catheter electrode is configured as a tip electrode disposed over a tip of the catheter and the first and third catheter electrodes are each configured as respective ring electrodes circumscribing a body of the catheter in a proximal direction in relation to the tip electrode.

5. The ablation system console of claim 1, the non-transitory computer readable medium further comprising instructions thereon, that when executed by the processor, cause the console to:
provide a first IRE ablation signal via the output port(s) as a first burst of biphasic pulses between the second and third catheter electrodes, and
provide a second IRE ablation signal via the output port(s) as a second burst of biphasic pulses between the first and third catheter electrodes.

6. The ablation system console of claim 5, the non-transitory computer readable medium further comprising instructions thereon, that when executed by the processor, cause the console to:
provide the first burst of biphasic pulses at an amplitude of about 900 V, and
provide the second burst of biphasic pulses at an amplitude of about 1800 V.

7. The ablation system console of claim 5, the non-transitory computer readable medium further comprising instructions thereon, that when executed by the processor, cause the console to:
provide a first RF ablation signal to the second catheter electrode via the output port(s),
provide a second RF ablation signal to the first catheter electrode via the output port(s),
simultaneously provide the first IRE ablation signal and the first RF ablation signal via the output port(s), and
simultaneously provide the second IRE ablation signal and the second RF ablation signal via the output port(s).

8. The ablation system console of claim 5, the non-transitory computer readable medium further comprising instructions thereon, that when executed by the processor, cause the console to:
provide the ablation energy via the output port(s) such that the first catheter electrode is configured as a tip electrode disposed over a tip of the catheter and the second and third catheter electrodes are each configured as respective ring electrodes circumscribing a body of the catheter in a proximal direction in relation to the tip electrode.

9. The ablation system console of claim 1, the non-transitory computer readable medium further comprising instructions thereon, that when executed by the processor, cause the console to:
provide the RF ablation signal to a first half of the first catheter electrode, the first half being electrically isolated from a second half of the first catheter electrode, the first and second halves disposed over a respective portion of a distal end of the catheter.

10. The ablation system console of claim 1, the non-transitory computer readable medium further comprising instructions thereon, that when executed by the processor, cause the console to:
determine a force vector applied to the distal portion of the catheter based on a sensor signal received by the console from the catheter.

11. The ablation system console of claim 1, further comprising:
an irrigation module configured to provide irrigation fluid to the first catheter electrode.

12. The ablation system console of claim 1, the non-transitory computer readable medium further comprising instructions thereon, that when executed by the processor, cause the console to:
determine temperature of the first catheter electrode based at least in part on a thermal sensor signal received from the catheter.

## Patentansprüche

1. Ablationssystemkonsole (40), umfassend:
einen oder mehrere Ausgangsanschlüsse (64), die konfiguriert sind, um einer ersten (102), einer zweiten (104) und einer dritten (106) Katheterelektrode Ablationsenergie bereitzustellen, während die erste, die zweite und die dritte Katheterelektrode auf einer Längsachse eines distalen Abschnitts eines Katheters (100) linear ausgerichtet sind;
einen Prozessor (32); und
ein nichtflüchtiges computerlesbares Medium in Kommunikation mit dem Prozessor und umfassend Anweisungen darauf, die, wenn sie durch den Prozessor ausgeführt werden, die Konsole veranlassen zum:
Bereitstellen eines HF-Ablationssignals über den Ausgangsanschluss/die Ausgangsanschlüsse an die erste Katheterelektrode,
Bereitstellen eines IRE-Ablationssignals über den Ausgangsanschluss/die Ausgangsanschlüsse als einen Burst biphasischer Impulse zwischen der zweiten und der dritten Katheterelektrode, und **dadurch gekennzeichnet, dass** das nichtflüchtige computerlesbare Medium ferner Anweisungen darauf umfasst, die, wenn sie durch den Prozessor ausgeführt werden, die Konsole veranlassen zum
Bereitstellen des Bursts biphasischer Impulse zwischen der zweiten Katheterelektrode und der dritten Katheterelektrode über den Ausgangsanschluss/die Ausgangsanschlüsse, während gleichzeitig das HF-Ablationssignal über den Ausgangsanschluss/die Ausgangsanschlüsse nur an die erste Katheterelektrode bereitgestellt wird.

2. Ablationssystemkonsole nach Anspruch 1, ferner umfassend:
einen Körperpflasteranschluss (69),
das nichtflüchtige computerlesbare Medium ferner umfassend Anweisungen darauf, die, wenn sie durch den Prozessor ausgeführt werden, die Konsole veranlassen zum:
Bereitstellen des IRE-Ablationssignals als einen Burst biphasischer Impulse zwischen einem Körperpflaster (65) über den Körperpflasteranschluss und mindestens einer der zweiten und der dritten Katheterelektrode über den Ausgangsanschluss/die Ausgangsanschlüsse.

3. Ablationssystemkonsole nach Anspruch 1, das nichtflüchtige computerlesbare Medium ferner umfassend Anweisungen darauf, die, wenn sie durch den Prozessor ausgeführt werden, den Prozessor veranlassen zum:
Bereitstellen des Bursts biphasischer Impulse und des HF-Ablationssignals über den Ausgangsanschluss/die Ausgangsanschlüsse derart, dass die erste Katheterelektrode als eine Spitzenelektrode konfiguriert ist, die über einer Spitze des Katheters angeordnet ist, und die zweite und die dritte Katheterelektrode jeweils als jeweilige Ringelektroden konfiguriert sind, die einen Körper des Katheters in einer proximalen Richtung in Bezug auf die Spitzenelektrode umgeben.

4. Ablationssystemkonsole nach Anspruch 1, das nichtflüchtige computerlesbare Medium ferner umfassend Anweisungen darauf, die, wenn sie durch den Prozessor ausgeführt werden, den Prozessor veranlassen zum:
Bereitstellen des Bursts biphasischer Impulse und des HF-Ablationssignals über den Ausgangsanschluss/die Ausgangsanschlüsse derart, dass die zweite Katheterelektrode als eine Spitzenelektrode konfiguriert ist, die über einer Spitze des Katheters angeordnet ist, und die erste und die dritte Katheterelektrode jeweils als jeweilige Ringelektroden konfiguriert sind, die einen Körper des Katheters in einer proximalen Richtung in Bezug auf die Spitzenelektrode umgeben.

5. Ablationssystemkonsole nach Anspruch 1, das nichtflüchtige computerlesbare Medium ferner umfassend Anweisungen darauf, die, wenn sie durch den Prozessor ausgeführt werden, den Prozessor veranlassen zum:
Bereitstellen eines ersten IRE-Ablationssignals über den Ausgangsanschluss/die Ausgangsanschlüsse als einen ersten Burst biphasischer Impulse zwischen der zweiten und der dritten Katheterelektrode, und
Bereitstellen eines zweiten IRE-Ablationssignals über den Ausgangsanschluss/die Ausgangsanschlüsse als ein zweiter Burst biphasischer Impulse zwischen der ersten und der dritten Katheterelektrode.

6. Ablationssystemkonsole nach Anspruch 5, das nichtflüchtige computerlesbare Medium ferner umfassend Anweisungen darauf, die, wenn sie durch den Prozessor ausgeführt werden, den Prozessor veranlassen zum:
Bereitstellen des ersten Bursts biphasischer Impulse mit einer Amplitude von etwa 900 V, und
Bereitstellen des zweiten Bursts biphasischer Impulse mit einer Amplitude von etwa 1800 V.

7. Ablationssystemkonsole nach Anspruch 5, das nichtflüchtige computerlesbare Medium ferner umfassend Anweisungen darauf, die, wenn sie durch den Prozessor ausgeführt werden, den Prozessor veranlassen zum:
Bereitstellen eines ersten HF-Ablationssignals an die zweite Katheterelektrode über den Ausgangsanschluss/die Ausgangsanschlüsse,
Bereitstellen eines zweiten HF-Ablationssignals an die erste Katheterelektrode über den Ausgangsanschluss/die Ausgangsanschlüsse,
gleichzeitiges Bereitstellen des ersten IRE-Ablationssignals und des ersten HF-Ablationssignals über den Ausgangsanschluss/die Ausgangsanschlüsse, und
gleichzeitiges Bereitstellen des zweiten IRE-Ablationssignals und des zweiten HF-Ablationssignals über den Ausgangsanschluss/die Ausgangsanschlüsse.

8. Ablationssystemkonsole nach Anspruch 5, das nichtflüchtige computerlesbare Medium ferner umfassend Anweisungen darauf, die, wenn sie durch den Prozessor ausgeführt werden, den Prozessor veranlassen zum:
Bereitstellen der Ablationsenergie über den Ausgangsanschluss/die Ausgangsanschlüsse derart, dass die erste Katheterelektrode als eine Spitzenelektrode konfiguriert ist, die über einer Spitze des Katheters angeordnet ist, und die zweite und die dritte Katheterelektrode jeweils als entsprechende Ringelektroden konfiguriert sind, die einen Körper des Katheters in einer proximalen Richtung in Bezug auf die Spitzenelektrode umgeben.

9. Ablationssystemkonsole nach Anspruch 1, das nichtflüchtige computerlesbare Medium ferner umfassend Anweisungen darauf, die, wenn sie durch den Prozessor ausgeführt werden, den Prozessor veranlassen zum:
Bereitstellen des HF-Ablationssignals an eine erste Hälfte der ersten Katheterelektrode, wobei die erste Hälfte von einer zweiten Hälfte der ersten Katheterelektrode elektrisch isoliert ist, wobei die erste und die zweite Hälfte über einem jeweiligen Abschnitt eines distalen Endes des Katheters angeordnet sind.

10. Ablationssystemkonsole nach Anspruch 1, das nichtflüchtige computerlesbare Medium ferner umfassend Anweisungen darauf, die, wenn sie durch den Prozessor ausgeführt werden, den Prozessor veranlassen zum:
Bestimmen eines Kraftvektors, der auf den distalen Abschnitt des Katheters ausgeübt wird, basierend auf einem Sensorsignal, das durch die Konsole von dem Katheter empfangen wird.

11. Ablationssystemkonsole nach Anspruch 1, ferner umfassend:
ein Spülmodul, das konfiguriert ist, um der ersten Katheterelektrode Spülflüssigkeit bereitzustellen.

12. Ablationssystemkonsole nach Anspruch 1, das nichtflüchtige computerlesbare Medium ferner umfassend Anweisungen darauf, die, wenn sie durch den Prozessor ausgeführt werden, den Prozessor veranlassen zum:
Bestimmen der Temperatur der ersten Katheterelektrode mindestens teilweise basierend auf einem thermischen Sensorsignal, das von dem Katheter empfangen wird.

## Revendications

1. Console de système d'ablation (40) comprenant :
un ou plusieurs ports de sortie (64) configurés pour fournir de l'énergie d'ablation à des première (102), deuxième (104) et troisième (106) électrodes de cathéter alors que les première, deuxième et troisième électrodes de cathéter sont alignées linéairement sur un axe longitudinal d'une partie distale d'un cathéter (100) ;
un processeur (32) ; et
un support non transitoire lisible par ordinateur en communication avec le processeur et comprenant des instructions sur celui-ci, qui lorsqu'elles sont exécutées par le processeur, amènent la console à :
fournir un signal d'ablation RF par l'intermédiaire du ou des port(s) de sortie à la première électrode de cathéter,
fournir un signal d'ablation IRE par l'intermédiaire du ou des port(s) de sortie en tant que salve d'impulsions biphasiques entre les deuxième et troisième électrodes de cathéter, et **caractérisée en ce que** le support non transitoire lisible par ordinateur comprend en outre des instructions sur celui-ci, qui lorsqu'elles sont exécutées par le processeur, amènent la console à
fournir la salve d'impulsions biphasiques entre la deuxième électrode de cathéter et la troisième électrode de cathéter par l'intermédiaire du ou des port(s) de sortie tout en fournissant simultanément le signal d'ablation RF uniquement à la première électrode de cathéter par l'intermédiaire du ou des port(s) de sortie.

2. Console de système d'ablation selon la revendication 1, comprenant en outre :
un port de patch corporel (69),
le support non transitoire lisible par ordinateur comprenant en outre des instructions sur celui-ci, qui lorsqu'elles sont exécutées par le processeur, amènent la console à :
fournir le signal d'ablation IRE en tant que salve d'impulsions biphasiques entre un patch corporel (65) par l'intermédiaire du port de patch corporel et au moins l'une des deuxième et troisième électrodes de cathéter par l'intermédiaire du ou des port(s) de sortie.

3. Console de système d'ablation selon la revendication 1, le support non transitoire lisible par ordinateur comprenant en outre des instructions sur celui-ci, qui lorsqu'elles sont exécutées par le processeur, amènent la console à :
fournir la salve d'impulsions biphasiques et le signal d'ablation RF par l'intermédiaire du ou des port(s) de sortie de telle sorte que la première électrode de cathéter est configurée en tant qu'électrode de pointe disposée par-dessus une pointe du cathéter et les deuxième et troisième électrodes de cathéter sont configurées chacune en tant qu'électrodes annulaires respectives circonscrivant un corps du cathéter dans une direction proximale par rapport à l'électrode de pointe.

4. Console de système d'ablation selon la revendication 1, le support non transitoire lisible par ordinateur comprenant en outre des instructions sur celui-ci, qui lorsqu'elles sont exécutées par le processeur, amènent la console à :
fournir la salve d'impulsions biphasiques et le signal d'ablation RF par l'intermédiaire du ou des port(s) de sortie de telle sorte que la deuxième électrode de cathéter est configurée en tant qu'électrode de pointe disposée par-dessus une pointe du cathéter et les première et troisième électrodes de cathéter sont configurées chacune en tant qu'électrodes annulaires respectives circonscrivant un corps du cathéter dans une direction proximale par rapport à l'électrode de pointe.

5. Console de système d'ablation selon la revendication 1, le support non transitoire lisible par ordinateur comprenant en outre des instructions sur celui-ci, qui lorsqu'elles sont exécutées par le processeur, amènent la console à :
fournir un premier signal d'ablation IRE par l'intermédiaire du ou des port(s) de sortie en tant que première salve d'impulsions biphasiques entre les deuxième et troisième électrodes de cathéter, et
fournir un second signal d'ablation IRE par l'intermédiaire du ou des port(s) de sortie en tant que seconde salve d'impulsions biphasiques entre les première et troisième électrodes de cathéter.

6. Console de système d'ablation selon la revendication 5, le support non transitoire lisible par ordinateur comprenant en outre des instructions sur celui-ci, qui lorsqu'elles sont exécutées par le processeur, amènent la console à :
fournir la première salve d'impulsions biphasiques à une amplitude d'environ 900 V, et
fournir la seconde salve d'impulsions biphasiques à une amplitude d'environ 1800 V.

7. Console de système d'ablation selon la revendication 5, le support non transitoire lisible par ordinateur comprenant en outre des instructions sur celui-ci, qui lorsqu'elles sont exécutées par le processeur, amènent la console à :
fournir un premier signal d'ablation RF à la deuxième électrode de cathéter par l'intermédiaire du ou des port(s) de sortie,
fournir un second signal d'ablation RF à la première électrode de cathéter par l'intermédiaire du ou des port(s) de sortie,
fournir simultanément le premier signal d'ablation IRE et le premier signal d'ablation RF par l'intermédiaire du ou des port(s) de sortie, et
fournir simultanément le second signal d'ablation IRE et le second signal d'ablation RF par l'intermédiaire du ou des port(s) de sortie.

8. Console de système d'ablation selon la revendication 5, le support non transitoire lisible par ordinateur comprenant en outre des instructions sur celui-ci, qui lorsqu'elles sont exécutées par le processeur, amènent la console à :
fournir l'énergie d'ablation par l'intermédiaire du ou des port(s) de sortie de telle sorte que la première électrode de cathéter est configurée en tant qu'électrode de pointe disposée par-dessus une pointe du cathéter et les deuxième et troisième électrodes de cathéter sont configurées chacune en tant qu'électrodes annulaires respectives circonscrivant un corps du cathéter dans une direction proximale par rapport à l'électrode de pointe.

9. Console de système d'ablation selon la revendication 1, le support non transitoire lisible par ordinateur comprenant en outre des instructions sur celui-ci, qui lorsqu'elles sont exécutées par le processeur, amènent la console à :
fournir le signal d'ablation RF à une première moitié de la première électrode de cathéter, la première moitié étant électriquement isolée d'une seconde moitié de la première électrode de cathéter, les première et seconde moitiés étant disposées par-dessus une partie respective d'une extrémité distale du cathéter.

10. Console de système d'ablation selon la revendication 1, le support non transitoire lisible par ordinateur comprenant en outre des instructions sur celui-ci, qui lorsqu'elles sont exécutées par le processeur, amènent la console à :
déterminer un vecteur de force appliqué à la partie distale du cathéter en fonction d'un signal de capteur reçu par la console en provenance du cathéter.

11. Console de système d'ablation selon la revendication 1, comprenant en outre :
un module d'irrigation configuré pour fournir un fluide d'irrigation à la première électrode de cathéter.

12. Console de système d'ablation selon la revendication 1, le support non transitoire lisible par ordinateur comprenant en outre des instructions sur celui-ci, qui lorsqu'elles sont exécutées par le processeur, amènent la console à :
déterminer la température de la première électrode de cathéter en fonction au moins en partie d'un signal de capteur thermique reçu du cathéter.
